(19) Europäisches Patentamt
European Patent Office
Office européen des brevets

(11) **EP 4 250 303 A1**

(12) **EUROPEAN PATENT APPLICATION**

(43) Date of publication:
**27.09.2023 Bulletin 2023/39**

(21) Application number: **22217113.4**

(22) Date of filing: **29.12.2022**

(51) International Patent Classification (IPC):
**G16C 20/30** (2019.01)     **G16C 20/40** (2019.01)
**G16B 15/00** (2019.01)

(52) Cooperative Patent Classification (CPC):
**G16C 20/30; G16B 15/00; G16C 20/40;**
G16C 10/00

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **22.03.2022   JP 2022045418**

(71) Applicant: **FUJITSU LIMITED**
**Kawasaki-shi, Kanagawa 211-8588 (JP)**

(72) Inventors:
• **Manabe, Toshio**
  **Kawasaki-shi, Kanagawa, 211-8588 (JP)**
• **Sato, Hiroyuki**
  **Kawasaki-shi, Kanagawa, 211-8588 (JP)**
• **Tanida, Yoshiaki**
  **Kawasaki-shi, Kanagawa, 211-8588 (JP)**
• **Terashima, Chieko**
  **Kawasaki-shi, Kanagawa, 211-8588 (JP)**

(74) Representative: **Hoffmann Eitle**
**Patent- und Rechtsanwälte PartmbB**
**Arabellastraße 30**
**81925 München (DE)**

(54) **SEARCH PROGRAM, SEARCH APPARATUS, AND METHOD OF SEARCHING FOR DETEMING A STABLE STRUCTURE OF COARSE-GRAINED MODEL OF A PEPTIDE**

(57)   A search program comprising instructions which, when executed by a computer, cause the computer to execute processing including: obtaining a first cost value of a first potential that corresponds to interaction between coarse-grained particles, a second cost value of a second potential that corresponds to an angle and a dihedral angle between the particles, and a third cost value of a third potential that corresponds to repulsion and attraction between the particles; and instructing an Ising apparatus to search for a structure of a coarse-grained model with which a total sum of the first cost value, the second cost value, and the third cost value calculated for an entirety of the coarse-grained model that includes a plurality of particles satisfies a predetermined condition.

FIG. 1

**Description**

FIELD

The embodiments discussed herein are related to a search program, a search apparatus, and a method of searching.

BACKGROUND

In recent years, in the field of drug discovery, drug discovery based on a middle molecule (having a molecular weight of 500 to 3000) that involves less side effects has been expected, and development of a method of searching to search for a stable structure of a middle molecule is in progress.

As an example, there is a method of searching in which, with respect to a coarse-grained model, a stable structure is searched by using an interaction potential between coarse-grained particles.

Examples of the related art include Japanese Laid-open Patent Publication Nos. 2019-185753 and 2021-192199.

SUMMARY

TECHNICAL PROBLEMS

However, in a case where the method of searching uses only the interaction potential between the coarse-grained particles as described above, an appropriate stable structure is not necessarily obtained for the coarse-grained model. Thus, improvement in search accuracy is desired.

In one aspect, an object is to improve search accuracy in searching for a stable structure of a coarse-grained model.

SOLUTION TO PROBLEM

According to an aspect of the embodiments, there is provided a search program comprising instructions which, when executed by a computer, cause the computer to execute processing including: obtaining a first cost value of a first potential that corresponds to interaction between coarse-grained particles, a second cost value of a second potential that corresponds to an angle and a dihedral angle between the particles, and a third cost value of a third potential that corresponds to repulsion and attraction between the particles; and instructing an Ising apparatus to search for a structure of a coarse-grained model with which a total sum of the first cost value, the second cost value, and the third cost value calculated for an entirety of the coarse-grained model that includes a plurality of particles satisfies a predetermined condition.

ADVANTAGEOUS EFFECTS OF INVENTION

The search accuracy in searching for a stable structure of a coarse-grained model may be improved.

BRIEF DESCRIPTION OF DRAWINGS

FIG. 1 is a first diagram exemplifying a system configuration of a stable structure search system and functional configurations of a terminal apparatus and an Ising apparatus;
FIG. 2 is a diagram illustrating an example of a hardware configuration of the terminal apparatus;
FIG. 3 is a diagram illustrating the details of a first potential information obtaining unit;
FIG. 4 is a diagram illustrating the details of second and third potential information obtaining units;
FIG. 5 is a diagram illustrating the details of fourth and fifth potential information obtaining units;
FIG. 6 is a diagram illustrating the details of a cost arithmetic expression generation unit;
FIG. 7 is a diagram illustrating the details of an evaluation unit and an example of an evaluation result;
FIG. 8 is a first flowchart illustrating a flow of a stable structure search process;
FIG. 9 is a second flowchart illustrating the flow of the stable structure search process;
FIG. 10 is a flowchart illustrating a flow of an adjustment process;
FIG. 11 is a second diagram exemplifying the system configuration of the stable structure search system and the functional configurations of the terminal apparatus and the Ising apparatus; and
FIG. 12 is a third flowchart illustrating the flow of the stable structure search process.

DESCRIPTION OF EMBODIMENTS

Each embodiment will be described below with reference to the accompanying drawings. In the present specification and drawings, elements having substantially the same functional configurations are denoted by the same numerals, thereby omitting redundant description.

[First Embodiment]

<System Configuration of Stable Structure Search System and Functional Configurations of Terminal Apparatus and Ising Apparatus>

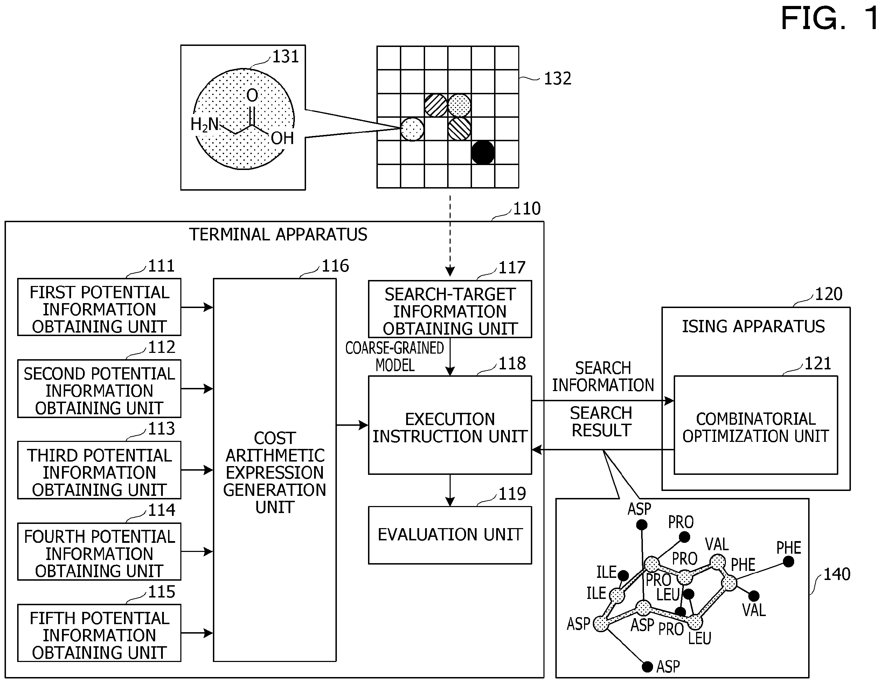

First, description will be given of a system configuration of a stable structure search system according to a first embodiment and functional configurations of a terminal apparatus and an Ising apparatus included in the stable structure search system. FIG. 1 is a first diagram exemplifying the system configuration of the stable structure search system and the functional configurations of the terminal apparatus and the Ising apparatus.

A stable structure search system 100 is a system for searching for a stable structure of a middle molecule and, for example, a system for searching for a stable structure of a cyclic peptide molecule having a plurality of amino acid residue sequences. For example, as a preliminary stage of searching for a stable structure of a cyclic peptide molecule, the stable structure search system 100 is a system that generates a coarse-grained model including a plurality of particles by replacing • a skeleton portion centered at an α carbon of an amino acid forming a main chain particle of a peptide and • a side chain particle with respective single coarse-grained particles (coarse-grained particles) and • disposing the particles in, for example, a space (lattice space) of a face-centered cubic (FCC) and the stable structure search system 100 searches for, as a combinatorial optimization problem, the stable structure of the generated coarse-grained model with the Ising apparatus.

Referring to FIG. 1, sign 131 indicates a state in which the skeleton portion centered at the α carbon of the amino acid included in the main chain particle of the peptide is replaced with a single coarse-grained particle. Sign 132 schematically indicates a state in which a plurality of coarse-grained particles (five coarse-grained particles in the example of FIG. 1) including the coarse-grained particle indicated by sign 131 are disposed in the FCC space (lattice space). For the sake of simplicity of illustration, in the example illustrated in FIG. 1, the FCC space is represented by a two dimensional plane.

A terminal apparatus 110 is an example of a search apparatus. The terminal apparatus 110 generates search information to be used when an Ising apparatus 120 searches for the stable structure of the coarse-grained model, transmits the search information to the Ising apparatus 120, receives a search result from the Ising apparatus 120, and evaluates the search result.

A search program is installed in the terminal apparatus 110. When this program is executed, the terminal apparatus 110 functions as first to fifth potential information obtaining units 111 to 115. The terminal apparatus 110 functions as a cost arithmetic expression generation unit 116, a search-target information obtaining unit 117, an execution instruction unit 118, and an evaluation unit 119.

Among these, the first potential information obtaining unit 111 obtains a Hamiltonian for calculating a cost value of the interaction potential of the entirety of the coarse-grained model and a Hamiltonian for calculating a cost value corresponding to the L-form and the D-form of the entirety of the coarse-grained model.

The first potential information obtaining unit 111 obtains a cost value of the interaction potential between the coarse-grained particles on a distance-by-distance basis with respect to distances between the coarse-grained particles. The first potential information obtaining unit 111 obtains a cost value corresponding to the difference between the L-form and the D-form of the coarse-grained particle.

The first potential information obtaining unit 111 notifies the cost arithmetic expression generation unit 116 of the obtained Hamiltonians and the obtained cost values.

The second potential information obtaining unit 112 obtains a Hamiltonian for calculating a cost value of a potential corresponding to an angle of the entirety of the coarse-grained model. The second potential information obtaining unit 112 obtains a cost value of a potential corresponding to an angle between the coarse-grained particles on an angle-by-angle basis with respect to angles between the coarse-grained particles.

The second potential information obtaining unit 112 notifies the cost arithmetic expression generation unit 116 of the obtained Hamiltonian and the obtained cost values.

The third potential information obtaining unit 113 obtains a Hamiltonian for calculating a cost value of a potential corresponding to a dihedral angle of the entirety of the coarse-grained model. The third potential information obtaining unit 113 obtains a cost value of a potential corresponding to the dihedral angle between the coarse-grained particles on a dihedral angle-by-dihedral angle basis with respect to dihedral angles between the coarse-grained particles.

The third potential information obtaining unit 113 notifies the cost arithmetic expression generation unit 116 of the obtained Hamiltonian and the obtained cost values.

The fourth potential information obtaining unit 114 obtains a Hamiltonian for calculating a cost value of a potential corresponding to repulsion (repulsive potential) of the entirety of the coarse-grained model. The fourth potential information obtaining unit 114 obtains, on a distance-by-distance basis with respect to the distances between the coarse-grained particles, a cost value of the repulsive potential which is a potential for correcting the interaction potential between the coarse-grained particles and which is a repulsive potential between the coarse-grained particles.

The fourth potential information obtaining unit 114 notifies the cost arithmetic expression generation unit 116 of the obtained Hamiltonian and the obtained cost values.

The fifth potential information obtaining unit 115 obtains a Hamiltonian for calculating a cost value of a potential corresponding to attraction (attractive potential) of the entirety of the coarse-grained model. The fifth potential information obtaining unit 115 obtains, on a distance-by-distance basis with respect to the distances between the coarse-grained particles, a cost value of the attractive potential which is a potential for correcting the interaction potential between the coarse-grained particles and which is an attractive potential between the coarse-grained particles.

The fifth potential information obtaining unit 115 notifies the cost arithmetic expression generation unit 116 of the obtained Hamiltonian and the obtained cost values.

The cost arithmetic expression generation unit 116 obtains the Hamiltonians and the cost values notified separately by the first to fifth potential information obtaining units 111 to 115. The cost arithmetic expression generation unit 116 combines the obtained Hamiltonians, thereby generating a cost arithmetic expression for calculating the total sum (total cost value) of the cost values of the potentials of the entirety of the coarse-grained model. The cost arithmetic expression generation unit 116 notifies the execution instruction unit 118 of the generated cost arithmetic expression and the obtained cost values.

The search-target information obtaining unit 117 obtains the search-target coarse-grained model, and the search-target information obtaining unit 117 notifies the execution instruction unit 118 of the obtained search-target coarse-grained model.

The execution instruction unit 118 transmits to the Ising apparatus 120 "search information" that includes • the cost arithmetic expression, • the cost values, and • the search-target coarse-grained model. Under each of the cost values, the execution instruction unit 118 instructs the Ising apparatus 120 to search for the stable structure with which the total cost value (the total sum of the cost values of the potentials) calculated, by using the cost arithmetic expression, for the entirety of the search-target coarse-grained model satisfies a predetermined condition.

The execution instruction unit 118 receives a search result and the like transmitted from the Ising apparatus 120 as a result of the transmission of the search information and notifies the evaluation unit 119 of the search result and the like. Examples of the search result and the like transmitted from the Ising apparatus 120 include, in addition to the stable structure of the coarse-grained model (search result), the following item: • search information transmitted to the Ising apparatus 120; • the total cost value that is calculated when the stable structure of the coarse-grained model is searched and that satisfies the predetermined condition; • a time (solution time) from the start of the search to the completion of the search when the stable structure of the coarse-grained model is searched; and the like. Sign 140 in FIG. 1 indicates a schematic example of the search result (stable structure of the coarse-grained model) transmitted from the Ising apparatus 120.

The evaluation unit 119 evaluates and outputs the search result and the like notified by the execution instruction unit 118. In evaluating the search result and the like, for an unknown search target, the evaluation unit 119 uses the total cost value that is calculated when the stable structure is searched and that satisfies the predetermined condition. Also in evaluating the search result and the like, for a known search target, the evaluation unit 119 uses a root-mean-square deviation (RMSD) of atomic positions calculated based on the searched stable structure and an actual structure (actually measured structure).

Meanwhile, an optimization program is installed in the Ising apparatus 120, and, when this program is executed, the Ising apparatus 120 functions as a combinatorial optimization unit 121.

Upon receiving the search information from the terminal apparatus 110, the combinatorial optimization unit 121 outputs a search result and the like by solving the stable structure of the coarse-grained model as a combinatorial optimization problem.

For example, under each of the cost values, the combinatorial optimization unit 121 searches for the stable structure with which the total cost value calculated, by using the cost arithmetic expression, for the entirety of the coarse-grained model as the search target satisfies the predetermined condition.

The combinatorial optimization unit 121 transmits a search result and the like including the searched stable structure (for example, sign 140) to the terminal apparatus 110.

As described above, in searching for the stable structure of the coarse-grained model, the stable structure search system 100 according to the first embodiment uses the total cost value for which, in addition to the interaction potential, potentials corresponding to the angle, the dihedral angle, the repulsion, and the attraction are taken into consideration.

Thus, according to the first embodiment, the search accuracy in searching for the stable structure of the coarse-grained model may be improved.

<Hardware Configuration of Terminal Apparatus>

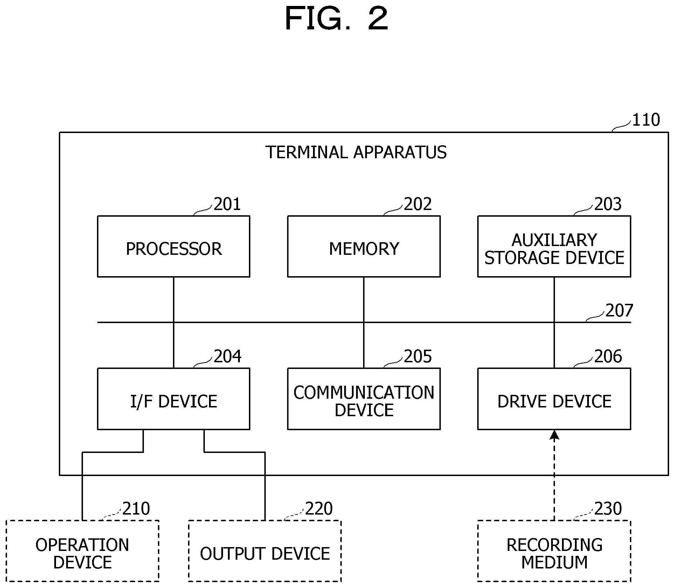

Next, a hardware configuration of the terminal apparatus 110 will be described. FIG. 2 is a diagram illustrating an example of the hardware configuration of the terminal apparatus.

As illustrated in FIG. 2, the terminal apparatus 110 includes a processor 201, a memory 202, an auxiliary storage device 203, an interface (I/F) device 204, a communication device 205, and a drive device 206. The various types of the hardware in the terminal apparatus 110 are coupled to each other via a bus 207.

The processor 201 includes various computing devices such as a central processing unit (CPU) and a graphics processing unit (GPU). The processor 201 loads various programs (for example, a search program and the like) onto the memory 202 and executes the programs.

The memory 202 includes main storage devices such as a read-only memory (ROM) and a random-access memory (RAM). The processor 201 and the memory 202 form a so-called computer. The computer realizes the above-described various functions when the processor 201 executes the various programs loaded onto the memory 202.

The auxiliary storage device 203 stores the various programs and various types of information to be used in execution of the various programs by the processor 201.

The I/F device 204 is a coupling device that allows coupling of an operation device 210 and the output device 220, which are examples of external devices, to the terminal apparatus 110.

The communication device 205 is a communication device for communicating with the Ising apparatus 120 via a network.

The drive device 206 is a device in which a recording medium 230 is to be set. Examples of the recording medium 230 herein include a medium on which information is recorded optically, electrically, or magnetically, such as a compact disc read-only memory (CD-ROM), a flexible disk, or a magneto-optical disk. Examples of the recording medium 230 may also include a semiconductor memory or the like on which information is recorded electrically, such as a ROM or a flash memory.

The various programs to be installed in the auxiliary storage device 203 are installed such that, for example, the distributed recording medium 230 is set in the drive device 206 and the drive device 206 reads the various programs recorded in the recording medium 230. Alternatively, the various programs to be installed in the auxiliary storage device 203 may be installed by being downloaded from the network via the communication device 205.

Although only the hardware configuration of the terminal apparatus 110 is described and the hardware configuration of the Ising apparatus 120 is not described herein, the hardware configuration of the Ising apparatus 120 may be similar to that of, for example, the terminal apparatus 110. Alternatively, the hardware configuration of the Ising apparatus 120 may be similar to the hardware configuration of a so-called quantum computer.

functional Configuration of Terminal Apparatus>

Next, the units realized in the terminal apparatus 110 (here, the first to fifth potential information obtaining units 111 to 115, the cost arithmetic expression generation unit 116, and the evaluation unit 119) will be described in detail.

(1) Details of First Potential Information Obtaining Unit

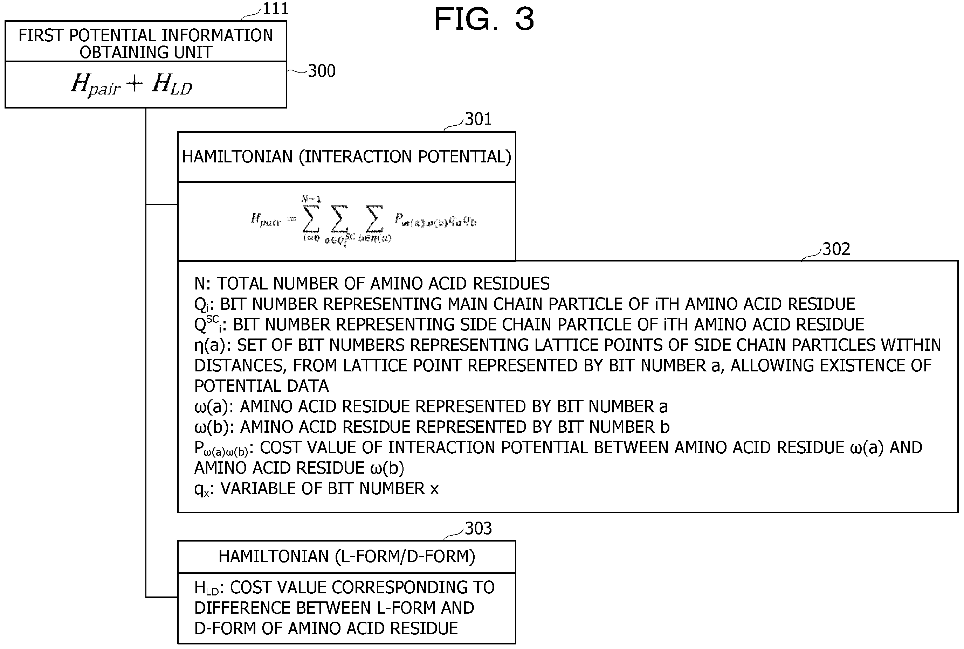

First, the details of the first potential information obtaining unit 111 are described. FIG. 3 is a diagram illustrating the details of the first potential information obtaining unit. In a formulation for solving the stable structure of the coarse-grained model as the combinatorial optimization problem with an annealing method, the first potential information obtaining unit 111 obtains the Hamiltonians represented by Expression (1) below.

$$H_{cost} = H_{pair} + H_{LD} \ldots \text{Expression (1)}$$

In Expression (1), $H_{pair}$ represents a Hamiltonian for calculating the cost value of the interaction potential of the entirety of the coarse-grained model. Also in Expression (1), $H_{LD}$ represents a Hamiltonian for calculating the cost value corresponding to the difference between the L-form and the D-form of the amino acid residue in the entirety of the coarse-grained model.

A Hamiltonian for calculating the cost value of the interaction potential of the entirety of the coarse-grained model may be represented by Expression (2) below.

$$H_{pair} = \sum_{i=0}^{N-1} \sum_{a \in Q_i^{SC}} \sum_{b \in \eta(a)} P_{\omega(a)\omega(b)} q_a q_b$$

... Expression (2)

In Expression (2) above, • N represents the total number of amino acid residues, • $Q_i$ represents a bit number representing the main chain of an ith amino acid residue, • $Q_i^{SC}$ represents a bit number representing the side chain particle of the ith amino acid residue, • $\eta(a)$ represents a set of bit numbers representing lattice points of the side chain particles within distances, from the lattice point represented by the bit number a, in which potential data exists, • $\omega(a)$ represents an amino acid residue represented by the bit number a, • $\omega(b)$ represents an amino acid residue represented by the bit number b, • $P_{\omega(a)\omega(b)}$ represents a cost value of an interaction potential between the amino acid residue $\omega(a)$ and the amino acid residue $\omega(b)$, and • $q_r$ represents a variable of the bit number x.

Among these, $P_{\omega(a)\omega(b)}$ is calculated in advance and organized into a library on an amino acid type-by-amino acid type basis. Thus, the first potential information obtaining unit 111 refers to the library to obtain the cost value of the interaction potential on a distance-by-distance basis with respect to distances between the amino acid residue $\omega(a)$ and the amino acid residue $\omega(b)$.

The first potential information obtaining unit 111 notifies the cost arithmetic expression generation unit 116 of the Hamiltonian indicated by sign 301 as the Hamiltonian for calculating the cost value of the interaction potential of the entirety of the coarse-grained model. The first potential information obtaining unit 111 notifies the cost arithmetic expression generation unit 116 of distance-by-distance $P_{\omega(a)\omega(b)}$ as the obtained distance-by-distance cost value of the interaction potential between the coarse-grained particles.

The first potential information obtaining unit 111 notifies the cost arithmetic expression generation unit 116 of the Hamiltonian indicated by sign 303 as the Hamiltonian for calculating the cost value corresponding to the difference between the L-form and the D-form of the entirety of the coarse-grained model. The first potential information obtaining unit 111 notifies the cost arithmetic expression generation unit 116 of the cost value corresponding to the difference between the L-form and the D-form of the amino acid residue.

(2) Details of Second and Third Potential Information Obtaining Units

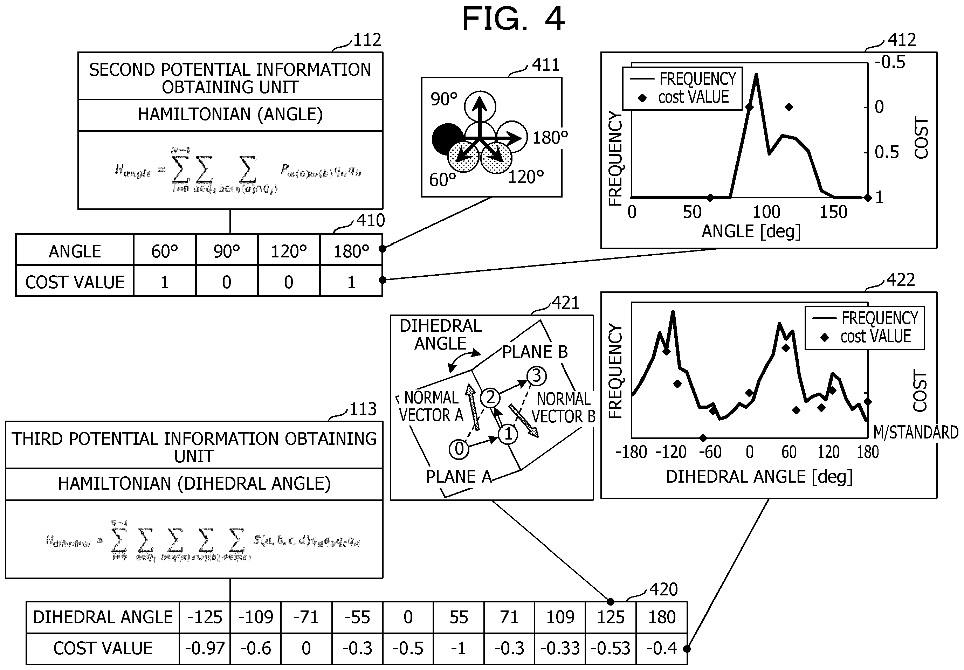

Next, the details of the second and third potential information obtaining units 112 and 113 are described. FIG. 4 is a diagram illustrating the details of the second and third potential information obtaining units. In the formulation for solving the stable structure of the coarse-grained model as the combinatorial optimization problem with the annealing method, the second potential information obtaining unit 112 obtains the Hamiltonian for calculating the cost value of the potential corresponding to the angle of the entirety of the coarse-grained model.

$$H_{angle} = \sum_{i=0}^{N-1} \sum_{a \in Q_i} \sum_{b \in (\eta(a) \cap Q_j)} P_{\omega(a)\omega(b)} q_a q_b$$

... Expression (3)

In Expression (3) above, $P_{\omega(a)\omega(b)}$ represents the angle-by-angle cost value with respect to the angles between the coarse-grained particles. Also in the above Expression (3), j = i + 2.

In a case where the lattice space in which the coarse-grained particles are disposed is an FCC space, an angle that may be formed between the coarse-grained particles is one of 60°, 90°, 120°, and 180° (sign 411 of FIG. 4). Accordingly, the second potential information obtaining unit 112 derives and obtains the angle-by-angle cost value with respect to the angles between the coarse-grained particles by • collecting structural data of a known molecular structure (for example, the molecular structure of a peptide) the features of which such as the number and the shape (whether the shape is an open-circular shape or a closed-circular shape) of the coarse-grained particles are relatively similar and • compiling the angle-by-angle appearance frequency of the collected structural data.

Referring to FIG. 4, sign 412 indicates a result of the compilation of the appearance frequency of the collected structural data in a case where the horizontal axis indicates an angle and the vertical axis indicates the appearance frequency. According to the sign 412, the appearance frequency is high in a case where the angle between the coarse-grained particles is 90° or 120° compared to a case where the angle between the coarse-grained particles is 60° or 180°.

The state "appearance frequency is high" indicates a structurally stable state, and the state "the appearance frequency is low" indicates a structurally unstable state. Accordingly, the second potential information obtaining unit 112 obtains a low cost value for an angle of "a high appearance frequency" and obtains a high cost value for an angle of "a low appearance frequency". Sign 410 of FIG. 4 indicates an example of the angle-by-angle cost value with respect to the angles between the coarse-grained particles obtained by the second potential information obtaining unit 112.

Likewise, in the formulation for solving the stable structure of the coarse-grained model as the combinatorial optimization problem with the annealing method, the third potential information obtaining unit 113 obtains the Hamiltonian for calculating the cost value of the potential corresponding to the dihedral angle of the entirety of the coarse-grained model.

$$H_{dihedral} = \sum_{i=0}^{N-1} \sum_{a \in Q_i} \sum_{b \in \eta(a)} \sum_{c \in \eta(b)} \sum_{d \in \eta(c)} S(a,b,c,d) q_a q_b q_c q_d$$

... Expression (4)

In Expression (4) above, S(a, b, c, d) represents the dihedral angle-by-dihedral angle cost value with respect to the dihedral angles between the coarse-grained particles.

Although the dihedral angle of the coarse-grained particle is calculated based on the positions of the four bonded main chain particles (i, i + 1, i + 2, i + 3) in the lattice space, the dihedral angle is desirably replaced with a quadratic expression that may be handled in the annealing method.

Thus, according to the present embodiment, auxiliary bits representing planes included in the dihedral angle are added, and the dihedral angle is represented by the combination of the auxiliary bits. For example, as indicated by sign 421, for the ith to (i + 2)th main chain particles forming an identical plane (a plane A), a normal vector A of the plane A is calculated from an outer product of • a vector from the ith main chain particle toward the (i + 1)th main chain particle and • a vector from the (i + 1)th main chain particle toward the (i + 2)th main chain particle, for the (i + 1)th to (i + 3)th main chain particles forming an identical plane (a plane B), a normal vector B of the plane B is calculated from an outer product of • a vector from the (i + 1)th main chain particle toward the (i + 2)th main chain particle and • a vector from the (i + 2)th main chain particle toward the (i + 3)th main chain particle, and the dihedral angle is calculated from an inner product of these two normal vectors (the normal vectors A and B).

The third potential information obtaining unit 113 derives and obtains the dihedral angle-by-dihedral angle cost value with respect to the dihedral angles between the coarse-grained particles by • collecting the structural data of a known molecular structure (for example, the molecular structure of a peptide) the features of which such as the number and the shape of the coarse-grained particles are relatively similar to the calculated dihedral angle and • compiling the dihedral angle-by-dihedral angle appearance frequencies of the collected structural data for the calculated dihedral angles.

Referring to FIG. 4, sign 422 indicates a result of the compilation of the appearance frequency of the collected structural data in a case where the horizontal axis indicates a dihedral angle and the vertical axis indicates the appearance frequency. According to sign 422, the appearance frequency significantly varies depending on the dihedral angle between the coarse-grained particles.

Accordingly, the third potential information obtaining unit 113 obtains, as the cost value, a negative value of the log of the appearance frequency. Sign 420 of FIG. 4 indicates examples of the dihedral angle-by-dihedral angle cost value between the coarse-grained particles obtained by the third potential information obtaining unit 113.

(3) Details of Fourth and Fifth Potential Information Obtaining Units

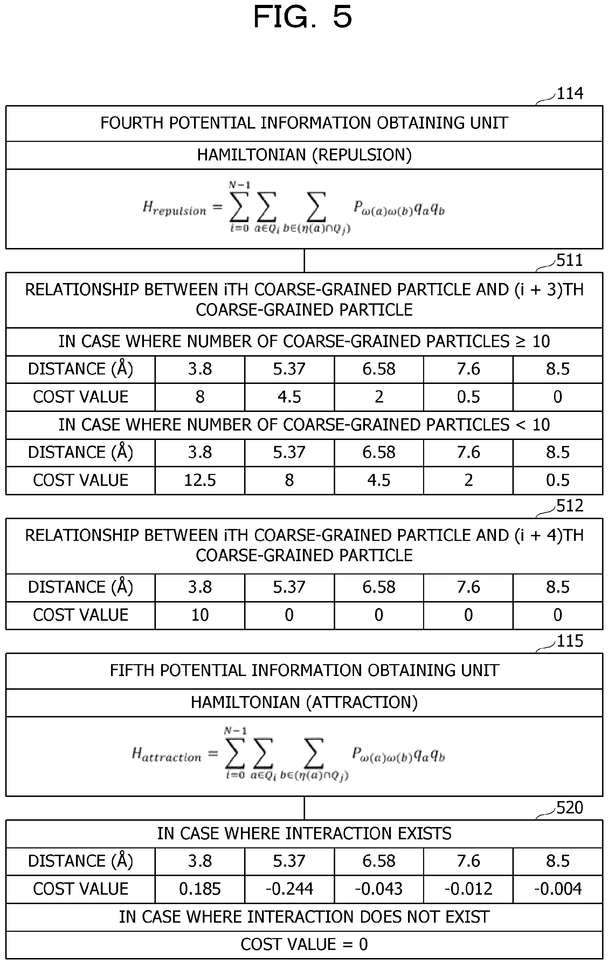

Next, the details of the fourth and fifth potential information obtaining units 114 and 115 are described. FIG. 5 is a diagram illustrating the details of the fourth and fifth potential information obtaining units.

In the formulation for solving the stable structure of the coarse-grained model as the combinatorial optimization problem with the annealing method, the fourth potential information obtaining unit 114 obtains the Hamiltonian for calculating the cost value of the repulsive potential of the entirety of the coarse-grained model.

$$H_{repulsion} = \sum_{i=0}^{N-1} \sum_{a \in Q_i} \sum_{b \in (\eta(a) \cap Q_j)} P_{\omega(a)\omega(b)} q_a q_b$$

... Expression (5)

In Expression (5) above, $P_{\omega(a)\omega(b)}$ represents the distance-by-distance cost value of the repulsive potential between the coarse-grained particles.

For description of the distance-by-distance cost value of the repulsive potential between coarse-grained particles, the relationship between the number and the shape of the coarse-grained particles is briefly described. In a case where the structure of a known peptide is analyzed, a cyclic peptide having a great number of amino acid residues (for example, the number of amino acid residues is 16) tends to have a linear shape, and the distance between the amino acid residues tends to increase. For example, when the distance between the ith amino acid residue and the (i + 3)th amino acid residue is analyzed for a cyclic peptide having 16 amino acid residues, the appearance frequency of 10 [Å] increases.

In contrast, a cyclic peptide having a small number of amino acid residues (for example, the number of amino acid residues is 8) tends to have a circular shape, and the distance between the amino acid residues tends to decrease. For example, when the distance between the ith amino acid residue and the (i + 3)th amino acid residue is analyzed for a cyclic peptide having 8 amino acid residues, the appearance frequency of 8.5 [Å] increases.

For example, in a case where the number of amino acid residues is great, it may be said that the (i + 3)th amino acid residue has a stable structure when the (i + 3)th amino acid is disposed at a position farther from the ith amino acid residue (a state in which the repulsion is great) compared to a case where the number of amino acid residues is small.

Accordingly, as the distance-by-distance cost value of the repulsive potential between the ith and (i + 3)th coarse-grained particles, the fourth potential information obtaining unit 114 obtains the cost values such that there is a difference in cost value between a case where the number of coarse-grained particles is greater than or equal to a predetermined threshold and in a case where the number of coarse-grained particles is smaller than the predetermined threshold.

Sign 511 of FIG. 5 indicates examples of cost values obtained by the fourth potential information obtaining unit 114. • These cost values are the distance-by-distance cost values of the repulsive potential between the ith coarse-grained particle and the (i + 3)th coarse-grained particle • in the case where the number of coarse-grained particles is greater than or equal to 10 and in the case where the number of coarse-grained particles is smaller than 10 (this applies to a case where j = i + 3 in Expression (5) above).

Similarly, in a case where the structure of a known peptide is analyzed, the distance between the ith amino acid residue and the (i + 4)th amino acid residue does not depend on the number of amino acid residues, and the appearance frequency of the distance smaller than the distance between the ith amino acid residue and the (i + 3)th amino acid residue increases. The reason for this is that, in the case of the (i + 4)th amino acid residue, the cases where the amino acid residues are disposed at opposite positions of the circular shape are increased.

Accordingly, as the distance-by-distance cost value of the repulsive potential between the ith and (i + 4)th coarse-grained particles, the fourth potential information obtaining unit 114 obtains a different cost value from the distance-by-distance cost value of the repulsive potential between the ith and (i + 3)th coarse-grained particles.

For example, the fourth potential information obtaining unit 114 obtains the cost values of the repulsive potential case-by-case as follows: • in a case where the distance between the ith coarse-grained particle and the (i + 4)th coarse-grained particle is smaller than a predetermined distance, the cost value increases as the distance decreases; and • in a case where the distance is greater than or equal to the predetermined distance, zero.

Sign 512 of FIG. 5 indicates examples of the distance-by-distance cost values of the repulsive potentials between the ith coarse-grained particle and the (i + 4)th coarse-grained particle obtained by the fourth potential information obtaining unit 114 (this applies to a case where j > i + 3 in Expression (5) above).

Similarly, in the formulation for solving the stable structure of the coarse-grained model as the combinatorial optimization problem with the annealing method, the fifth potential information obtaining unit 115 obtains the Hamiltonian for calculating the cost value of the attractive potential of the entirety of the coarse-grained model.

$$H_{attraction} = \sum_{i=0}^{N-1} \sum_{a \in Q_i} \sum_{b \in (\eta(a) \cap Q_j)} P_{\omega(a)\omega(b)} q_a q_b$$

... Expression (6)

In Expression (6) above, $P_{\omega(a)\omega(b)}$ represents the distance-by-distance cost value of the attractive potential between the coarse-grained particles.

For example, the fifth potential information obtaining unit 115 obtains the cost value of the attractive potential applied when it is presumed that interaction such as a hydrogen bond acts between molecules of the coarse-grained particles. Also, the fifth potential information obtaining unit 115 obtains a constraint that ensures a bond between the coarse-grained particles set in a case where a clear bond such as an S-S bond exists between the coarse-grained particles.

Sign 520 of FIG. 5 indicates examples of the distance-by-distance cost values of the attractive potentials which are cost values obtained by the fifth potential information obtaining unit 115 and applied in a case where the interaction acts between the molecules of the coarse-grained particles. As indicated by sign 520, the cost value of the attractive potential which is a cost value obtained by the fifth potential information obtaining unit 115 in a case where no interaction acts between the molecules of the coarse-grained particles is zero. The cost value in either case is applied in a case where j > i + 3 in Expression (6) above (where i and j are designated).

(6) Details of Cost Arithmetic Expression Generation Unit

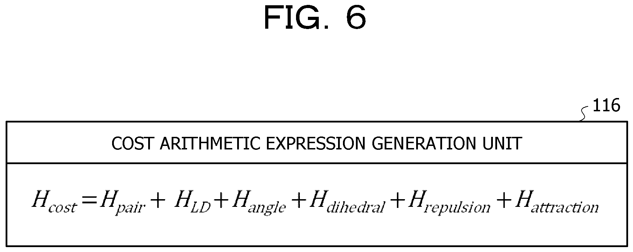

Next, the details of the cost arithmetic expression generation unit 116 are described. FIG. 6 is a diagram illustrating the details of the cost arithmetic expression generation unit. As illustrated in FIG. 6, according to the present embodiment, the following cost values are given between bits representing coordinates in the lattice space of the coarse-grained particles, and thereby the cost arithmetic expression generation unit 116 combines the Hamiltonians together to generate Expression (7) as the cost arithmetic expression that realizes the calculation of the total cost value: • the distance-by-distance cost value of the interaction potential between the coarse-grained particles and the cost value corresponding to the difference between the L-form and the D-form; • the angle-by-angle cost value of the potential corresponding to the angle between the coarse-grained particles; • the dihedral angle-by-dihedral angle cost value of the potential corresponding to the dihedral angle between the coarse-grained particles; • the distance-by-distance cost value of the repulsive potential between the coarse-grained particles; and • the distance-by-distance cost value of the attractive potential between the coarse-grained particles.

$$H_{cost} = H_{pair} + H_{LD} + H_{angle} + H_{dihedral} + H_{repulsion} + H_{attraction} \text{ ... Expression (7)}$$

Also, the cost arithmetic expression generation unit 116 notifies the execution instruction unit 118 of the following expression and the cost values as the search information: • the generated cost arithmetic expression; • the distance-by-distance cost value of the interaction potential between the coarse-grained particles and the cost value corresponding to the difference between the L-form and the D-form; • the angle-by-angle cost value of the potential corresponding to the angle between the coarse-grained particles (sign 410); • the dihedral angle-by-dihedral angle cost value of the potential corresponding to the dihedral angle between the coarse-grained particles (sign 420); • the distance-by-distance cost value of the repulsive potential between the coarse-grained particles (signs 511 and 512); and • the distance-by-distance cost value of the attractive potential between the coarse-grained particles (sign 520).

Accordingly, the execution instruction unit 118 adds the coarse-grained model notified by the search-target information obtaining unit 117 to the search information notified by the cost arithmetic expression generation unit 116, transmits the search information and the coarse-grained model to the Ising apparatus 120, and instructs the Ising apparatus to search for a stable structure.

(7) Details of Evaluation Unit and Evaluation Result

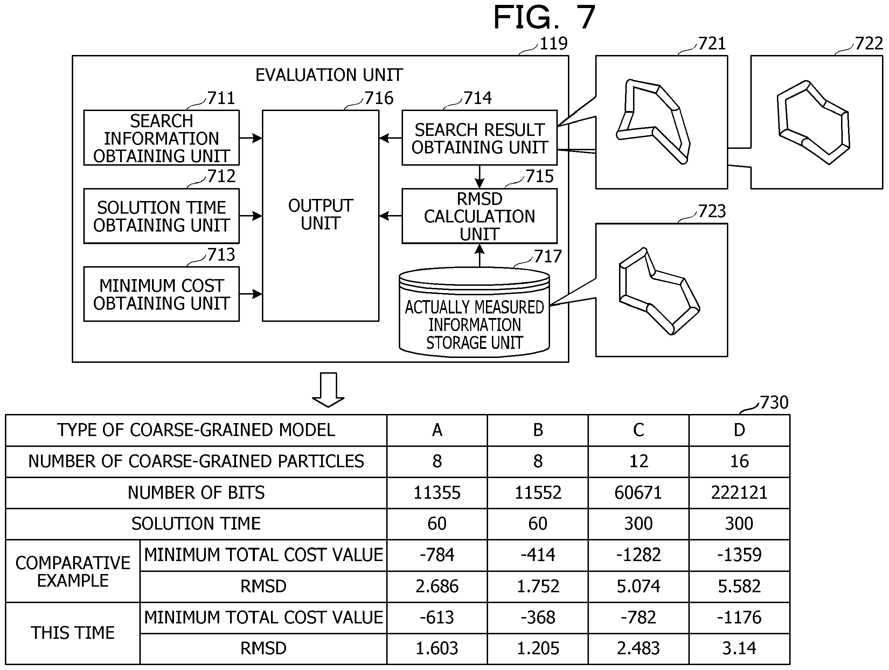

Next, the details of the evaluation unit 119 and the evaluation result are described. FIG. 7 is a diagram illustrating the details of the evaluation unit and an example of an evaluation result. As illustrated in FIG. 7, the evaluation unit 119 includes a search information obtaining unit 711, a solution time obtaining unit 712, a minimum cost obtaining unit 713, a search result obtaining unit 714, an RMSD calculation unit 715, and an output unit 716.

The search information obtaining unit 711 obtains the search information that the execution instruction unit 118 transmits to the Ising apparatus 120 when the execution instruction unit 118 instructs the Ising apparatus 120 to search for the stable structure of the coarse-grained model.

The solution time obtaining unit 712 obtains search time from a time at which the execution instruction unit 118 instructs the Ising apparatus 120 to search for the stable structure of the coarse-grained model to a time at which the search result is obtained.

The minimum cost obtaining unit 713 obtains the total cost value that is calculated when the Ising apparatus 120 searches for the stable structure and that satisfies the predetermined condition.

The search result obtaining unit 714 obtains the search result output by the Ising apparatus 120. Signs 721 and 722 in FIG. 7 indicate examples of the obtained search result.

In a case where the search target is a known coarse-grained model, the RMSD calculation unit 715 calculates the RMSD based on • the search result obtained by the search result obtaining unit 714 and • the actual measurement information corresponding to the search result (actually measured structure stored in an actually measured information storage unit 717 in advance (for example, sign 723)).

The output unit 716 outputs information obtained or calculated in the units included in the evaluation unit 119 as an evaluation result. An evaluation result 730 illustrated in FIG. 7 is an example of the evaluation result output by the output unit 716.

As illustrated in FIG. 7, the evaluation result 730 output by the output unit 716 includes, as information items, "TYPE OF COARSE-GRAINED MODEL", "NUMBER OF COARSE-GRAINED PARTICLES", "NUMBER OF BITS", "SOLUTION TIME", "COMPARATIVE EXAMPLE", and "THIS TIME".

Information indicating the type of the coarse-grained model is stored in "TYPE OF COARSE-GRAINED MODEL". The example of FIG. 7 illustrates that the evaluation unit 119 has evaluated four types of coarse-grained models.

The number of coarse-grained particles included in the corresponding coarse-grained model is stored in "NUMBER OF COARSE-GRAINED PARTICLES". The example of FIG. 7 illustrates that the coarse-grained models of the types = "A" and "B" are coarse-grained models in which the number of coarse-grained particles is eight, and the coarse-grained models of the types = "C" and "D" are coarse-grained models in which the number of coarse-grained particles is 16.

The amount of calculation in searching for the stable structure for the corresponding coarse-grained model is stored in "NUMBER OF BITS". The solution time in searching for the stable structure for the corresponding coarse-grained model is stored in "SOLUTION TIME".

The minimum total cost value and the RMSD for its solution in a case where the stable structure is searched for corresponding coarse-grained model by using Expression (1) above as the cost arithmetic expression are stored in "COMPARATIVE EXAMPLE".

The minimum total cost value and the RMSD for its solution in a case where the stable structure is searched for corresponding coarse-grained model by using Expression (7) above as the cost arithmetic expression are stored in "THIS TIME".

As indicated in the evaluation result 730, in any one of the coarse-grained models, the RMSD for the solution of the minimum total cost value is a smaller value in this time than in the comparative example. For example, with the stable structure search system 100 according to the first embodiment, the search accuracy in searching for the stable structure of the coarse-grained model may be improved.

Although "TYPE OF COARSE-GRAINED MODEL", "NUMBER OF COARSE-GRAINED PARTICLES", "NUMBER OF BITS", "SOLUTION TIME", "COMPARATIVE EXAMPLE", and "THIS TIME" are output as the information items of the evaluation result in the example illustrated in FIG. 7, the information items to be output as the evaluation result are not limited to these. For example, the search result (signs 721 and 722), the actually measured structure (sign 723), or the like may be output as the evaluation result.

<Flow of Stable Structure Search Process>

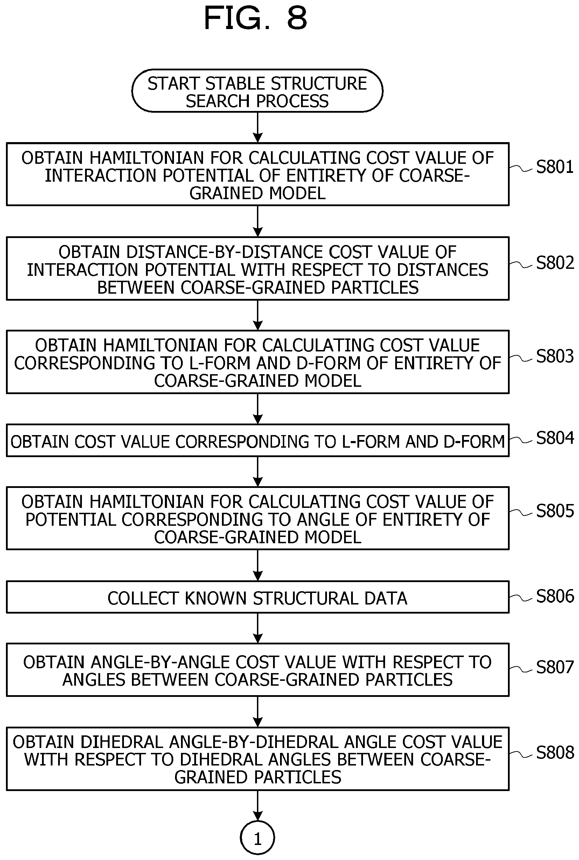

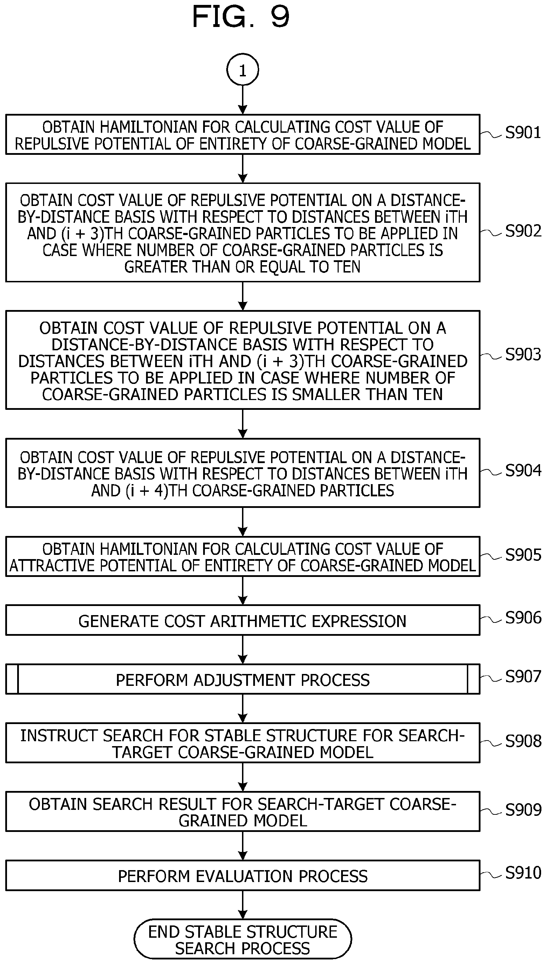

Next, a flow of a stable structure search process performed by the terminal apparatus 110 is described. FIGs. 8 and 9 are respectively first and second flowcharts illustrating the flow of the stable structure search process.

In step S801, the terminal apparatus 110 obtains the Hamiltonian for calculating the cost value of the interaction potential of the entirety of the coarse-grained model.

In step S802, the terminal apparatus 110 obtains the distance-by-distance cost value of the interaction potential with respect to the distances between the coarse-grained particles.

In step S803, the terminal apparatus 110 obtains the Hamiltonian for calculating the cost value corresponding to the L-form and the D-form of the entirety of the coarse-grained model.

In step S804, the terminal apparatus 110 obtains the cost value corresponding to the L-form and the D-form of the coarse-grained particle.

In step S805, the terminal apparatus 110 obtains the Hamiltonian for calculating the cost value of the potential corresponding the angle of the entirety of the coarse-grained model.

In step S806, the terminal apparatus 110 collects structural data of a known peptide the features of which such as the number and shape of coarse-grained particles included in the search-target coarse-grained model are relatively similar.

In step S807, the terminal apparatus 110 compiles the angle-by-angle appearance frequency with respect to the collected structural data, thereby obtaining the angle-by-angle cost value with respect to the angles between the coarse-grained particles.

In step S808, the terminal apparatus 110 compiles the dihedral angle-by-dihedral angle appearance frequency with respect to the collected structural data, thereby obtaining the dihedral angle-by-dihedral angle cost value with respect to the dihedral angles between the coarse-grained particles.

Next, in step S901 illustrated in FIG. 9, the terminal apparatus 110 obtains the Hamiltonian for calculating the cost value of the repulsive potential of the entirety of the coarse-grained model.

In step S902, the terminal apparatus 110 obtains the cost value of the repulsive potential on a distance-by-distance basis with respect to the distances between the ith and the (i + 3)th coarse-grained particles to be applied in the case where the number of the coarse-grained particles is greater than or equal to ten.

In step S903, the terminal apparatus 110 obtains the cost value of the repulsive potential on a distance-by-distance basis with respect to the distances between the ith and (i + 3)th coarse-grained particles to be applied in the case where the number of the coarse-grained particles is smaller than ten.

In step S904, the terminal apparatus 110 obtains the cost value of the repulsive potential on a distance-by-distance basis with respect to the distances between the ith and the (i + 4)th coarse-grained particles to be applied independently of the number of coarse-grained particles.

In step S905, the terminal apparatus 110 obtains the Hamiltonian for calculating the cost value of the attractive potential of the entirety of the coarse-grained model.

In step S906, the terminal apparatus 110 combines the obtained Hamiltonians together, thereby generating the cost arithmetic expression for calculating the total cost value of the entirety of the coarse-grained model.

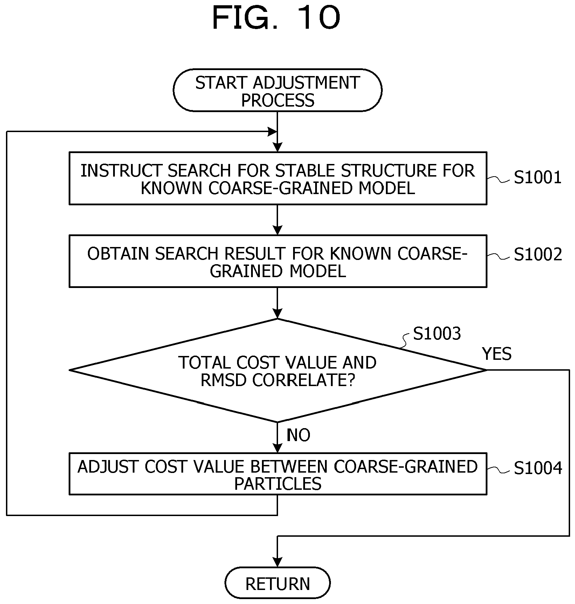

In step S907, the terminal apparatus 110 performs an adjustment process for adjusting the cost value between the coarse-grained particles by using a known coarse-grained model. The details of the adjustment process will be described later with reference to FIG. 10.

In step S908, the terminal apparatus 110 instructs search for the stable structure for the search-target coarse-grained model. To instruct the search, the terminal apparatus 110 transmits to the Ising apparatus 120 the search information that includes the cost arithmetic expression, each of the cost values, and the search-target coarse-grained model.

In step S909, the terminal apparatus 110 obtains the search result and the like for the search-target coarse-grained model from the Ising apparatus 120.

In step S910, the terminal apparatus 110 evaluates the stable structure of the search-target coarse-grained model by using the obtained search result and the like and outputs the evaluation result.

<Details of Adjustment Process>

Next, the adjustment process illustrated in FIG. 9 (step S907) is described in detail. FIG. 10 is a flowchart illustrating a flow of the adjustment process.

In step S1001, the terminal apparatus 110 instructs search for the stable structure for a known coarse-grained model.

In step S1002, the terminal apparatus 110 obtains the search result and the like for the known coarse-grained model from the Ising apparatus 120.

In step S1003, the terminal apparatus 110 evaluates the obtained search result and the like and determines whether the total cost value and the RMSD correlate.

In step S1003, in a case where it is determined that the total cost value and the RMSD do not correlate, (in the case of NO in step S1003), the process proceeds to step S1004.

After adjusting the cost value between the coarse-grained particles in step S1004, the terminal apparatus 110 returns to step S1001.

In contrast, in a case where it is determined that the total cost value and the RMSD correlate in step S1003 (in the case of YES in step S1003), the process ends the adjustment process and returns to step S908 illustrated in FIG. 9.

As is clearly understood from the above description, the terminal apparatus 110 according to the first embodiment obtains the following cost values: • the cost value of the interaction potential between the coarse-grained particles; • the cost value of the potential corresponding to the angle and the dihedral angle between the coarse-grained particles, and • the cost value of the potential corresponding to the repulsion and the attraction between the coarse-grained particles.

Under each of the obtained cost values, the terminal apparatus 110 according to the first embodiment instructs the Ising apparatus to search for the structure of the coarse-grained model with which the total sum of the cost values (total cost value) of the potentials calculated for the entirety of the coarse-grained model satisfies the predetermined condition.

As described above, in searching for the stable structure of the coarse-grained model, the terminal apparatus 110 according to the first embodiment instructs the search of the stable structure performed by using the total cost value for which, in addition to the interaction potential, potentials corresponding to the angle, the dihedral angle, the repulsion, and the attraction are taken into consideration.

Thus, according to the first embodiment, the search accuracy in searching for the stable structure of the coarse-grained model may be improved.

[Second Embodiment]

According to the above-described first embodiment, the process up to the evaluation of the search result and the like has been described. In contrast, according to a second embodiment, a process up to execution of molecular dynamics calculation using the search result and the like will be described. The second embodiment will be described by focusing on the differences from the above-described first embodiment.

<System Configuration of Stable Structure Search System and Functional Configurations of Terminal Apparatus and Ising Apparatus>

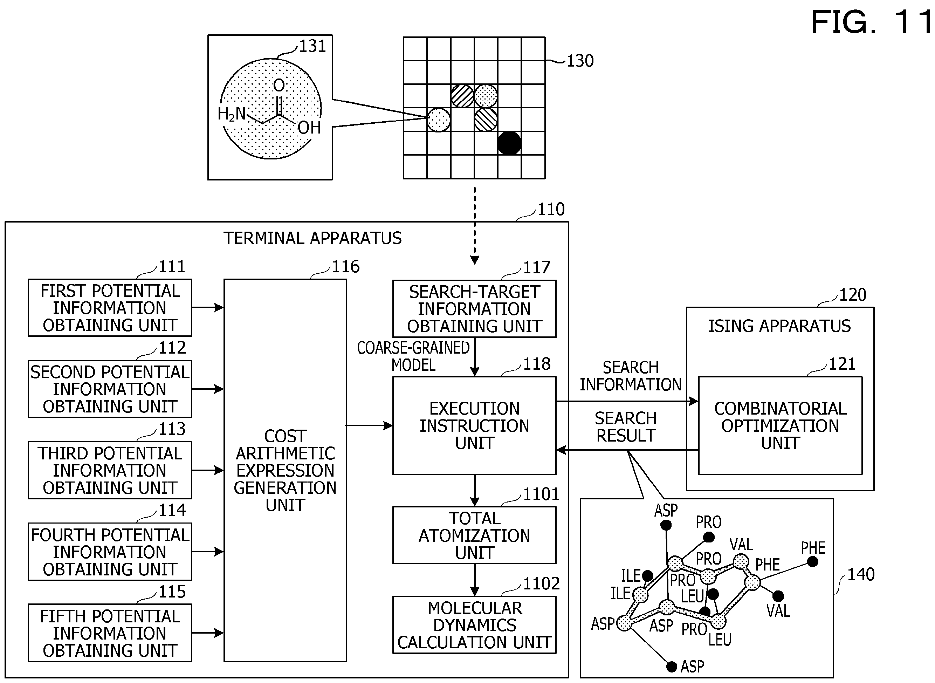

First, description will be given of a system configuration of the stable structure search system according to the second embodiment and the functional configurations of the terminal apparatus and the Ising apparatus included in the stable structure search system. FIG. 11 is a second diagram exemplifying the system configuration of the stable structure search system and the functional configurations of the terminal apparatus and the Ising apparatus. The difference between the first embodiment described above and the second embodiment is that the terminal apparatus 110 includes a total atomization unit 1101 and a molecular dynamics calculation unit 1102 instead of the evaluation unit 119.

The total atomization unit 1101 obtains the search result (coarse-grained model having a stable structure) received by the execution instruction unit 118 and performs total atomization on the main chain particle. Also, the total atomization unit 1101 inputs the search result in which the total atomization has been performed on the main chain particle to the molecular dynamics calculation unit 1102 as an initial structure to perform a molecular dynamics calculation process.

The molecular dynamics calculation unit 1102 performs the molecular dynamics calculation process by using the initial structure input by the total atomization unit 1101.

<Flow of Stable Structure Search Process>

Next, a flow of the stable structure search process performed by the terminal apparatus 110 is described. Also in the stable structure search process according to the second embodiment, it is assumed that the process illustrated in FIG. 8 is executed in a similar manner to that of the above-described first embodiment. It is also assumed that the process illustrated in FIG. 9 is executed in a different manner from that of the above-described first embodiment.

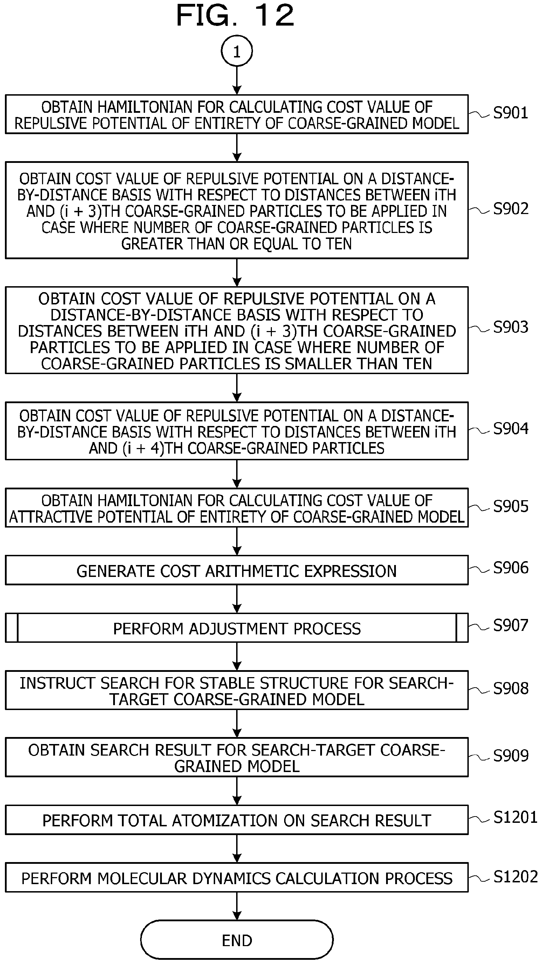

FIG. 12 is a third flowchart illustrating the flow of the stable structure search process. FIG. 12 corresponds to the second flowchart (FIG. 9) described in the above-described first embodiment. The difference between FIG. 9 and FIG. 12 is that, in the case of FIG. 12, steps S1201 and S1202 are added instead of the step S910.

In step S1201, the terminal apparatus 110 performs total atomization on the main chain particles of the search result (coarse-grained model having a stable structure).

In step S1202, the terminal apparatus 110 performs the molecular dynamics calculation process with the search result in which the total atomization has been performed on the main chain particles as the initial structure.

As is clearly understood from the above description, the terminal apparatus 110 according to the second embodiment has, in addition to the functions of the terminal apparatus 110 according to the first embodiment, the functions of performing the total atomization on the main chain particles of the search result and inputting, as the initial structure of the molecular dynamics calculation process, the search result in which the total atomization has been performed on the main chain particles.

Thus, according to the second embodiment, similar effects to those of the above-described first embodiment may be obtained and an appropriate molecular dynamics calculation process may be executed.

[Other Embodiments]

Each of the embodiments above is described on the assumption that the stable structure search system 100 is formed by the terminal apparatus 110 and the Ising apparatuses 120. However, the stable structure search system 100 may be formed by an apparatus other than the terminal apparatus 110 and the Ising apparatus 120 (for example, three or more apparatuses). Alternatively, the stable structure search system 100 may be formed by an apparatus made by integrating the terminal apparatus 110 and the Ising apparatus 120 with each other (for example, a single apparatus).

The first embodiment above has been described on the assumption that the terminal apparatus 110 includes the first potential information obtaining unit 111 to the evaluation unit 119 and the Ising apparatus 120 includes the combinatorial optimization unit 121. However, a subset of the functions of the terminal apparatus 110 may be realized by the Ising apparatus 120. Likewise, a subset of the functions of the Ising apparatus 120 may be realized by the terminal apparatus 110.

Modes as in the appendixes to be described below are conceivable according to the disclosed technique.

The present disclosure is not limited to the configurations illustrated herein but may include configurations such as a combination of any of the configurations exemplified in the above-described embodiments with other elements. These aspects may be changed without departing from the gist of the present disclosure and appropriately set in accordance with application modes thereof.

**Claims**

1. A search program comprising instructions which, when executed by a computer, cause the computer to execute processing comprising:

   obtaining a first cost value of a first potential that corresponds to interaction between coarse-grained particles, a second cost value of a second potential that corresponds to an angle and a dihedral angle between the particles, and a third cost value of a third potential that corresponds to repulsion and attraction between the particles; and
   instructing an Ising apparatus to search for a structure of a coarse-grained model with which a total sum of the first cost value, the second cost value, and the third cost value calculated for an entirety of the coarse-grained model that includes a plurality of particles satisfies a predetermined condition.

2. The search program according to claim 1, wherein

   the second cost value of the second potential includes
   an angle-by-angle fourth cost value, with respect to angles between the particles, that is derived from structural data of a known molecular structure based on an appearance frequency of angles formed between main chain particles, and
   a dihedral angle-by-dihedral angle fifth cost value, with respect to dihedral angles between the particles, that is derived from the structural data of the known molecular structure based on an appearance frequency of dihedral angles formed between the main chain particles.

3. The search program according to claim 1, wherein

   the third cost value of the third potential includes
   a distance-by-distance sixth cost value of a potential that corresponds to repulsion between an ith particle and an (i + 3)th particle.

4. The search program according to claim 3, wherein
   the sixth cost value varies depending on a number of the plurality of particles.

5. The search program according to claim 1, wherein

   the third cost value of the third potential includes
   a distance-by-distance seventh cost value of a potential that corresponds to repulsion between an ith particle and an (i + 4)th particle.

6. The search program according to claim 1, wherein

   the third cost value of the third potential includes
   a distance-by-distance eighth cost value of a potential that corresponds to the attraction between the particles and that is applied in a case where it is determined that interaction between the particles exists.

7. The search program according to claim 1, the process further comprising:

obtaining a first Hamiltonian for calculating the first cost value for the entirety of the coarse-grained model, a second Hamiltonian for calculating the second cost value for the entirety of the coarse-grained model, and a third Hamiltonian for calculating the third cost value for the entirety of the coarse-grained model; and instructing the Ising apparatus to calculate the total sum by using the first Hamiltonian, the second Hamiltonian, and the third Hamiltonian.

8. The search program according to claim 1, the process further comprising:
obtaining, in accordance with the instruction to the Ising apparatus, as a search result, the structure of the coarse-grained model with which the total sum satisfies the predetermined condition.

9. The search program according to claim 8, the process further comprising:

obtaining, as the search result, a structure of the coarse-grained model with which the total sum is minimized; and performing total atomization on the main chain particles to output the obtained structure as an initial structure of a molecular dynamics calculation process.

10. A search apparats comprising:

an obtaining unit that obtains a first cost value of a first potential that corresponds to interaction between coarse-grained particles, a second cost value of a second potential that corresponds to an angle and a dihedral angle between the particles, and a third cost value of a third potential that corresponds to repulsion and attraction between the particles; and
an instructing unit that instructs an Ising apparatus to search for a structure of a coarse-grained model with which a total sum of the first cost value, the second cost value, and the third cost value calculated for an entirety of the coarse-grained model that includes a plurality of particles satisfies a predetermined condition.

11. A search method implemented by a computer, the search method comprising:

obtaining a first cost value of a first potential that corresponds to interaction between coarse-grained particles, a second cost value of a second potential that corresponds to an angle and a dihedral angle between the particles, and a third cost value of a third potential that corresponds to repulsion and attraction between the particles; and
instructing an Ising apparatus to search for a structure of a coarse-grained model with which a total sum of the first cost value, the second cost value, and the third cost value calculated for an entirety of the coarse-grained model that includes a plurality of particles satisfies a predetermined condition.

FIG. 1

EP 4 250 303 A1

# FIG. 2

# FIG. 3

**FIRST POTENTIAL INFORMATION OBTAINING UNIT** /111

$$H_{pair} + H_{LD}$$ /300

**HAMILTONIAN (INTERACTION POTENTIAL)** /301

$$H_{pair} = \sum_{i=0}^{N-1} \sum_{a \in Q_i^{SC}} \sum_{b \in \eta(a)} P_{\omega(a)\omega(b)} q_a q_b$$

/302

N: TOTAL NUMBER OF AMINO ACID RESIDUES

$Q_i$: BIT NUMBER REPRESENTING MAIN CHAIN PARTICLE OF iTH AMINO ACID RESIDUE

$Q^{SC}_i$: BIT NUMBER REPRESENTING SIDE CHAIN PARTICLE OF iTH AMINO ACID RESIDUE

$\eta(a)$: SET OF BIT NUMBERS REPRESENTING LATTICE POINTS OF SIDE CHAIN PARTICLES WITHIN DISTANCES, FROM LATTICE POINT REPRESENTED BY BIT NUMBER a, ALLOWING EXISTENCE OF POTENTIAL DATA

$\omega(a)$: AMINO ACID RESIDUE REPRESENTED BY BIT NUMBER a

$\omega(b)$: AMINO ACID RESIDUE REPRESENTED BY BIT NUMBER b

$P_{\omega(a)\omega(b)}$: COST VALUE OF INTERACTION POTENTIAL BETWEEN AMINO ACID RESIDUE $\omega(a)$ AND AMINO ACID RESIDUE $\omega(b)$

$q_x$: VARIABLE OF BIT NUMBER x

**HAMILTONIAN (L-FORM/D-FORM)** /303

$H_{LD}$: COST VALUE CORRESPONDING TO DIFFERENCE BETWEEN L-FORM AND D-FORM OF AMINO ACID RESIDUE

# FIG. 4

SECOND POTENTIAL INFORMATION OBTAINING UNIT — 112

HAMILTONIAN (ANGLE)

$$H_{angle} = \sum_{i=0}^{N-1} \sum_{a \in Q_i} \sum_{b \in (\eta(a) \cap Q_j)} P_{\omega(a)\omega(b)} q_a q_b$$

411

| ANGLE | 60° | 90° | 120° | 180° |
|---|---|---|---|---|
| COST VALUE | 1 | 0 | 0 | 1 |

410

412

THIRD POTENTIAL INFORMATION OBTAINING UNIT — 113

HAMILTONIAN (DIHEDRAL ANGLE)

$$H_{dihedral} = \sum_{i=0}^{N-1} \sum_{a \in Q_i} \sum_{b \in \eta(a)} \sum_{c \in \eta(b)} \sum_{d \in \eta(c)} S(a,b,c,d) q_a q_b q_c q_d$$

421

422

| DIHEDRAL ANGLE | -125 | -109 | -71 | -55 | 0 | 55 | 71 | 109 | 125 | 180 |
|---|---|---|---|---|---|---|---|---|---|---|
| COST VALUE | -0.97 | -0.6 | 0 | -0.3 | -0.5 | -1 | -0.3 | -0.33 | -0.53 | -0.4 |

420

EP 4 250 303 A1

# FIG. 5

<table>
<tr><td colspan="6">FOURTH POTENTIAL INFORMATION OBTAINING UNIT</td></tr>
<tr><td colspan="6">HAMILTONIAN (REPULSION)</td></tr>
<tr><td colspan="6">$$H_{repulsion} = \sum_{i=0}^{N-1} \sum_{a \in Q_i} \sum_{b \in (\eta(a) \cap Q_j)} P_{\omega(a)\omega(b)} q_a q_b$$</td></tr>
</table>

114

<table>
<tr><td colspan="6">RELATIONSHIP BETWEEN iTH COARSE-GRAINED PARTICLE AND (i + 3)TH COARSE-GRAINED PARTICLE</td></tr>
<tr><td colspan="6">IN CASE WHERE NUMBER OF COARSE-GRAINED PARTICLES ≥ 10</td></tr>
<tr><td>DISTANCE (Å)</td><td>3.8</td><td>5.37</td><td>6.58</td><td>7.6</td><td>8.5</td></tr>
<tr><td>COST VALUE</td><td>8</td><td>4.5</td><td>2</td><td>0.5</td><td>0</td></tr>
<tr><td colspan="6">IN CASE WHERE NUMBER OF COARSE-GRAINED PARTICLES < 10</td></tr>
<tr><td>DISTANCE (Å)</td><td>3.8</td><td>5.37</td><td>6.58</td><td>7.6</td><td>8.5</td></tr>
<tr><td>COST VALUE</td><td>12.5</td><td>8</td><td>4.5</td><td>2</td><td>0.5</td></tr>
</table>

511

<table>
<tr><td colspan="6">RELATIONSHIP BETWEEN iTH COARSE-GRAINED PARTICLE AND (i + 4)TH COARSE-GRAINED PARTICLE</td></tr>
<tr><td>DISTANCE (Å)</td><td>3.8</td><td>5.37</td><td>6.58</td><td>7.6</td><td>8.5</td></tr>
<tr><td>COST VALUE</td><td>10</td><td>0</td><td>0</td><td>0</td><td>0</td></tr>
</table>

512

<table>
<tr><td colspan="6">FIFTH POTENTIAL INFORMATION OBTAINING UNIT</td></tr>
<tr><td colspan="6">HAMILTONIAN (ATTRACTION)</td></tr>
<tr><td colspan="6">$$H_{attraction} = \sum_{i=0}^{N-1} \sum_{a \in Q_i} \sum_{b \in (\eta(a) \cap Q_j)} P_{\omega(a)\omega(b)} q_a q_b$$</td></tr>
</table>

115

<table>
<tr><td colspan="6">IN CASE WHERE INTERACTION EXISTS</td></tr>
<tr><td>DISTANCE (Å)</td><td>3.8</td><td>5.37</td><td>6.58</td><td>7.6</td><td>8.5</td></tr>
<tr><td>COST VALUE</td><td>0.185</td><td>-0.244</td><td>-0.043</td><td>-0.012</td><td>-0.004</td></tr>
<tr><td colspan="6">IN CASE WHERE INTERACTION DOES NOT EXIST</td></tr>
<tr><td colspan="6">COST VALUE = 0</td></tr>
</table>

520

# FIG. 6

116

| COST ARITHMETIC EXPRESSION GENERATION UNIT |
| --- |
| $$H_{cost} = H_{pair} + H_{LD} + H_{angle} + H_{dihedral} + H_{repulsion} + H_{attraction}$$ |

# FIG. 7

| TYPE OF COARSE-GRAINED MODEL | | A | B | C | D |
|---|---|---|---|---|---|
| NUMBER OF COARSE-GRAINED PARTICLES | | 8 | 8 | 12 | 16 |
| NUMBER OF BITS | | 11355 | 11552 | 60671 | 222121 |
| SOLUTION TIME | | 60 | 60 | 300 | 300 |
| COMPARATIVE EXAMPLE | MINIMUM TOTAL COST VALUE | -784 | -414 | -1282 | -1359 |
| | RMSD | 2.686 | 1.752 | 5.074 | 5.582 |
| THIS TIME | MINIMUM TOTAL COST VALUE | -613 | -368 | -782 | -1176 |
| | RMSD | 1.603 | 1.205 | 2.483 | 3.14 |

EP 4 250 303 A1

# FIG. 8

START STABLE STRUCTURE
SEARCH PROCESS

OBTAIN HAMILTONIAN FOR CALCULATING COST VALUE OF
INTERACTION POTENTIAL OF ENTIRETY OF COARSE-
GRAINED MODEL — S801

OBTAIN DISTANCE-BY-DISTANCE COST VALUE OF
INTERACTION POTENTIAL WITH RESPECT TO DISTANCES
BETWEEN COARSE-GRAINED PARTICLES — S802

OBTAIN HAMILTONIAN FOR CALCULATING COST VALUE
CORRESPONDING TO L-FORM AND D-FORM OF ENTIRETY OF
COARSE-GRAINED MODEL — S803

OBTAIN COST VALUE CORRESPONDING TO L-FORM AND D-FORM — S804

OBTAIN HAMILTONIAN FOR CALCULATING COST VALUE OF
POTENTIAL CORRESPONDING TO ANGLE OF ENTIRETY OF
COARSE-GRAINED MODEL — S805

COLLECT KNOWN STRUCTURAL DATA — S806

OBTAIN ANGLE-BY-ANGLE COST VALUE WITH RESPECT TO
ANGLES BETWEEN COARSE-GRAINED PARTICLES — S807

OBTAIN DIHEDRAL ANGLE-BY-DIHEDRAL ANGLE COST VALUE
WITH RESPECT TO DIHEDRAL ANGLES BETWEEN COARSE-
GRAINED PARTICLES — S808

( 1 )

# FIG. 9

( 1 )

OBTAIN HAMILTONIAN FOR CALCULATING COST VALUE OF REPULSIVE POTENTIAL OF ENTIRETY OF COARSE-GRAINED MODEL — S901

OBTAIN COST VALUE OF REPULSIVE POTENTIAL ON A DISTANCE-BY-DISTANCE BASIS WITH RESPECT TO DISTANCES BETWEEN iTH AND (i + 3)TH COARSE-GRAINED PARTICLES TO BE APPLIED IN CASE WHERE NUMBER OF COARSE-GRAINED PARTICLES IS GREATER THAN OR EQUAL TO TEN — S902

OBTAIN COST VALUE OF REPULSIVE POTENTIAL ON A DISTANCE-BY-DISTANCE BASIS WITH RESPECT TO DISTANCES BETWEEN iTH AND (i + 3)TH COARSE-GRAINED PARTICLES TO BE APPLIED IN CASE WHERE NUMBER OF COARSE-GRAINED PARTICLES IS SMALLER THAN TEN — S903

OBTAIN COST VALUE OF REPULSIVE POTENTIAL ON A DISTANCE-BY-DISTANCE BASIS WITH RESPECT TO DISTANCES BETWEEN iTH AND (i + 4)TH COARSE-GRAINED PARTICLES — S904

OBTAIN HAMILTONIAN FOR CALCULATING COST VALUE OF ATTRACTIVE POTENTIAL OF ENTIRETY OF COARSE-GRAINED MODEL — S905

GENERATE COST ARITHMETIC EXPRESSION — S906

PERFORM ADJUSTMENT PROCESS — S907

INSTRUCT SEARCH FOR STABLE STRUCTURE FOR SEARCH-TARGET COARSE-GRAINED MODEL — S908

OBTAIN SEARCH RESULT FOR SEARCH-TARGET COARSE-GRAINED MODEL — S909

PERFORM EVALUATION PROCESS — S910

END STABLE STRUCTURE SEARCH PROCESS

# FIG. 10

START ADJUSTMENT PROCESS

INSTRUCT SEARCH FOR STABLE STRUCTURE FOR KNOWN COARSE-GRAINED MODEL — S1001

OBTAIN SEARCH RESULT FOR KNOWN COARSE-GRAINED MODEL — S1002

S1003

TOTAL COST VALUE AND RMSD CORRELATE? — YES

NO

ADJUST COST VALUE BETWEEN COARSE-GRAINED PARTICLES — S1004

RETURN

FIG. 11

# FIG. 12

(1)

OBTAIN HAMILTONIAN FOR CALCULATING COST VALUE OF REPULSIVE POTENTIAL OF ENTIRETY OF COARSE-GRAINED MODEL ~ S901

OBTAIN COST VALUE OF REPULSIVE POTENTIAL ON A DISTANCE-BY-DISTANCE BASIS WITH RESPECT TO DISTANCES BETWEEN iTH AND (i + 3)TH COARSE-GRAINED PARTICLES TO BE APPLIED IN CASE WHERE NUMBER OF COARSE-GRAINED PARTICLES IS GREATER THAN OR EQUAL TO TEN ~ S902

OBTAIN COST VALUE OF REPULSIVE POTENTIAL ON A DISTANCE-BY-DISTANCE BASIS WITH RESPECT TO DISTANCES BETWEEN iTH AND (i + 3)TH COARSE-GRAINED PARTICLES TO BE APPLIED IN CASE WHERE NUMBER OF COARSE-GRAINED PARTICLES IS SMALLER THAN TEN ~ S903

OBTAIN COST VALUE OF REPULSIVE POTENTIAL ON A DISTANCE-BY-DISTANCE BASIS WITH RESPECT TO DISTANCES BETWEEN iTH AND (i + 4)TH COARSE-GRAINED PARTICLES ~ S904

OBTAIN HAMILTONIAN FOR CALCULATING COST VALUE OF ATTRACTIVE POTENTIAL OF ENTIRETY OF COARSE-GRAINED MODEL ~ S905

GENERATE COST ARITHMETIC EXPRESSION ~ S906

PERFORM ADJUSTMENT PROCESS ~ S907

INSTRUCT SEARCH FOR STABLE STRUCTURE FOR SEARCH-TARGET COARSE-GRAINED MODEL ~ S908

OBTAIN SEARCH RESULT FOR SEARCH-TARGET COARSE-GRAINED MODEL ~ S909

PERFORM TOTAL ATOMIZATION ON SEARCH RESULT ~ S1201

PERFORM MOLECULAR DYNAMICS CALCULATION PROCESS ~ S1202

END

Europäisches
Patentamt

European
Patent Office

Office européen
des brevets

# EUROPEAN SEARCH REPORT

Application Number

EP 22 21 7113

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/383897 A1 (TERASHIMA CHIEKO [JP] ET AL) 9 December 2021 (2021-12-09) * the whole document * | 1-11 | INV. G16C20/30 G16C20/40 G16B15/00 |
| X | FOGOLARI FEDERICO ET AL: "Scoring predictive models using a reduced representation of proteins: model and energy definition", BMC STRUCTURAL BIOLOGY, BIOMED CENTRAL LTD., LONDON, GB, vol. 7, no. 1, 23 March 2007 (2007-03-23), page 15, XP021023639, ISSN: 1472-6807, DOI: 10.1186/1472-6807-7-15 * the whole document * | 1-11 | |
| X | ANWAR RAYAN ET AL: "Exploring the conformational space of cyclic peptides by a stochastic search method", JOURNAL OF MOLECULAR GRAPHICS AND MODELLING, vol. 22, no. 5, 1 May 2004 (2004-05-01), pages 319-333, XP055692382, US ISSN: 1093-3263, DOI: 10.1016/j.jmgm.2003.12.012 * the whole document * | 1-11 | TECHNICAL FIELDS SEARCHED (IPC) G16C G16B |
| X | ANTON ROBERT ET AL: "Resource-Efficient Quantum Algorithm for Protein Folding", ARXIV.ORG, CORNELL UNIVERSITY LIBRARY, 201 OLIN LIBRARY CORNELL UNIVERSITY ITHACA, NY 14853, 6 August 2019 (2019-08-06), XP081893186, DOI: 10.1038/S41534-021-00368-4 * the whole document * | 1-11 | |

The present search report has been drawn up for all claims

| Place of search | Date of completion of the search | Examiner |
|---|---|---|
| The Hague | 20 July 2023 | Denoual, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 21 7113

This annex lists the patent family members relating to the patent documents cited in the above-mentioned European search report.
The members are as contained in the European Patent Office EDP file on
The European Patent Office is in no way liable for these particulars which are merely given for the purpose of information.

20-07-2023

| Patent document cited in search report | | Publication date | Patent family member(s) | | Publication date |
|---|---|---|---|---|---|
| US 2021383897 | A1 | 09-12-2021 | CN | 113764053 A | 07-12-2021 |
| | | | EP | 3920187 A1 | 08-12-2021 |
| | | | JP | 2021192199 A | 16-12-2021 |
| | | | US | 2021383897 A1 | 09-12-2021 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019185753 A

- JP 2021192199 A